# EUROPEAN PATENT SPECIFICATION

(11) **EP 4 333 867 B1**
(45) Date of publication and mention of the grant of the patent: **18.12.2024**
(21) Application number: 22719598.9
(22) Date of filing: 03.05.2022
(51) Int. Cl.: A61K 38/10, A61K 39/00, C07K 7/00, C07K 7/08, C07K 14/005

(54) **PEPTIDES INHIBITING COVID-19**
COVID-19-BINDENDES PEPTID
PEPTIDE DE LIAISON COVID-19

(30) Priority: 03.05.2021 EP 21171841; 28.05.2021 EP 21176473
(43) Date of publication of application: 13.03.2024
(73) Proprietor: QuantBioRes A/S, 2860 Søborg (DK)
(72) Inventor: COSIC, Irena, Black Rock, Victoria 3193 (AU); COSIC, Drasko, Black Rock, Victoria 3193 (AU)
(74) Representative: Høiberg P/S
(86) International application number: PCT/EP2022/061825
(87) International publication number: WO 2022/233856

(56) References cited:
- ANNA-WINONA STRUCK ET AL: "A hexapeptide of the receptor-binding domain of SARS corona virus spike protein blocks viral entry into host cells via the human receptor ACE2", ANTIVIRAL RESEARCH, ELSEVIER BV, NL, vol. 94, no. 3, 20 December 2011 (2011-12-20), pages 288 - 296, XP028505925, ISSN: 0166-3542, [retrieved on 20120117], DOI: 10.1016/J.ANTIVIRAL.2011.12.012
- EGIEYEH SAMUEL ET AL: "Computational drug repurposing strategy predicted peptide-based drugs that can potentially inhibit the interaction of SARS-CoV-2 spike protein with its target (humanACE2)", PLOS ONE, vol. 16, no. 1, 8 January 2021 (2021-01-08), pages e0245258, XP055842632, DOI: 10.1371/journal.pone.0245258
- SOUZA PEDRO F N ET AL: "A molecular docking study revealed that synthetic peptides induced conformational changes in the structure of SARS-CoV-2 spike glycoprotein, disrupting the interaction with human ACE2 receptor", INTERNATIONAL JOURNAL OF BIOLOGICAL MACROMOLECULES, ELSEVIER BV, NL, vol. 164, 18 July 2020 (2020-07-18), pages 66 - 76, XP086335577, ISSN: 0141-8130, [retrieved on 20200718], DOI: 10.1016/J.IJBIOMAC.2020.07.174
- SHRAVANB RATHOD ET AL: "Peptide modelling and screening against human ACE2 and spike glycoprotein RBD of SARS-CoV-2", IN SILICO PHARMACOLOGY, BIOMED CENTRAL LTD, LONDON, UK, vol. 8, no. 1, 9 November 2020 (2020-11-09), pages 1 - 9, XP021284007, DOI: 10.1007/S40203-020-00055-W
- PATTNAIK GOURAB PRASAD ET AL: "Entry Inhibitors: Efficient Means to Block Viral Infection", JOURNAL OF MEMBRANE BIOLOGY, SPRINGER NEW YORK LLC, US, vol. 253, no. 5, 30 August 2020 (2020-08-30), pages 425 - 444, XP037256256, ISSN: 0022-2631, [retrieved on 20200830], DOI: 10.1007/S00232-020-00136-Z
- VANPATTEN SONYA ET AL: "Evidence supporting the use of peptides and peptidomimetics as potential SARS-CoV-2 (COVID-19) therapeutics", vol. 12, no. 18, 16 July 2020 (2020-07-16), GB, pages 1647 - 1656, XP055842614, ISSN: 1756-8919, Retrieved from the Internet <URL:https://www.ncbi.nlm.nih.gov/pmc/articles/PMC7364852/pdf/fmc-2020-0180.pdf> DOI: 10.4155/fmc-2020-0180

## Description

### Technical field

The present invention relates to novel peptides which can block the binding interaction of novel coronavirus SARS-CoV-2 in mammals, specifically by inhibiting the interaction between the spike protein of coronaviruses and the ACE2 receptor. The invention further relates to said peptides for use in treatment of COVID-19. Even further, the present invention relates to the peptides of the invention for use in inducing an immune response in a mammal by administration of said peptides.

### Background

Historically, infectious diseases, particularly due to viruses, have been one of the most important contributors to human morbidity and mortality. They account for a large proportion of death and disability worldwide and remain in certain regions the most important cause of ill health. The burden of viral diseases and disorders is illustrated by the recent outbreak of coronavirus disease 2019 (COVID-19).

COVID-19 is an infectious disease caused by the recently discovered coronavirus Severe Acute Respiratory Syndrome CoronaVirus 2 (SARS-CoV-2), often referred to as the COVID-19 virus. Viral airway infection COVID-19 is also referred to as 2019-nCoV acute respiratory disease (2019-nCoV ARD) and novel coronavirus pneumonia (NCP).

Most people infected with the COVID-19 virus will experience mild to moderate respiratory illness resembling mild pneumonia and recover without requiring special treatment. Older people, and those with underlying medical problems like cardiovascular disease, diabetes, chronic respiratory disease, and cancer are more likely to develop serious illness. Common symptoms include fever, tiredness, and dry cough. Other symptoms include shortness of breath, aches and pains and sore throat.

The clinical spectrum of COVID-19 varies from asymptomatic to clinical conditions characterized by respiratory failure that necessitates mechanical ventilation and intensive care unit support, to multiorgan and systemic manifestations in terms of sepsis, septic shock, and multiple organ dysfunction syndromes.

With the latest outbreak of coronavirus, and with the number of previous corona RNA virus outbreaks like SARS and MERS, there is a question if it is possible to design universal coronavirus vaccines or even a cure. So far vaccines are based on immune response to certain protein or DNA/RNA sequences within the virus. As many viruses, particularly RNA viruses have extremely variable genome sequence, such approach is not always successful. For example, there is still not generally successful vaccine for HIV virus and in the case of influenza, virus vaccines exist for each strain separately. If it is possible to identify common components of proteins responsible for the initial infection for all corona related RNA viruses and then design a vaccine or treatment based on such components, we will be able to design a general vaccine or treatment and thereby prevention of all analysed coronaviruses.

The current COVID-19 pandemic has caused havoc in the worlds, including the number of medical problems from mild symptoms up to severe and critical symptoms, and even death, in addition to the enormous economic problems associated with worldwide lockdown. The main problem is that this pandemic was caused by a new, hereto unknown virus, now classified as SARS-CoV-2, which triggered fear how this highly infectious and unknown virus will affect human population. The SARS-CoV-2 virus belongs to the group of coronaviruses, which are RNA viruses. Coronaviruses are widely spread in nature, mostly infecting animals, but some are capable of infecting humans as well, usually with mild or non-existent symptoms. However, there have been so far three instances where coronavirus has infected humans, via animals, and causing severe problems, namely SARS (Severe Acute Respiratory Syndrome, 2003), MERS (Middle East Respiratory Syndrome, 2012) and COVID-19 (SARS-CoV-2).

As SARS-CoV-2 is a completely new virus, there is an urgent need to investigate all aspects of SARS-CoV-2 virus infection and its activity.

### Summary

The invention is as defined in the claims.

The present inventors have designed novel peptides, which are capable of blocking the interaction between the angiotensin-converting enzyme 2 (ACE2) receptor found on cell surfaces, and the Spike-S1 protein of the SARS-CoV-2 coronavirus.

Thus, in an aspect, the present invention concerns a peptide, wherein the peptide comprises an amino acid sequence selected from the group consisting of SEQ ID NO: 1, SEQ ID NO: 2, SEQ ID NO: 3, SEQ ID NO: 4, SEQ ID NO: 5, and SEQ ID NO: 6, or a sequence variant thereof differing by one or two amino acid residues from said SEQ ID NO.

The novel peptides of the invention may be formulated as part of a composition for administration to a subject. The composition may also be formulated to comprising polynucleotides, vectors, host cells and/or other means of delivering the peptides of the invention to a subject.

Thus, in another aspect, the present invention concerns a composition comprising the peptides of the invention.

In yet another aspect, the present invention concerns a polynucleotide encoding the peptides of the invention.

In yet another aspect, the present invention concerns a vector comprising a polynucleotide encoding the peptides of the invention.

In yet another aspect, the present invention concerns a host cell comprising the polynucleotide encoding the peptides of the invention, or the vector comprising said polynucleotide.

By studying the *in vitro* properties of the novel peptides towards the ACE2/Spike-S1 interaction associated with COVID19 infections in humans, it was found that 80% of ACE2/Spike-S1 binding events may be avoided by implementing the novel peptides for use in treatment of COVID19.

In a second aspect, the present invention concerns the peptide, the composition, the polynucleotide, the vector, and/or the host cell as disclosed above and defined in the claims, for use as a medicament.

In a third aspect, the present invention concerns the peptide, the composition, the polynucleotide, the vector, and/or the host cell as disclosed above and defined in the claims, for use in prevention and/or treatment of a SARS-CoV-2-related disease in a subject.

In a fourth aspect, the present invention concerns the peptide, the composition, the polynucleotide, the vector, and/or the host cell as disclosed above and defined in the claims, for inducing an immune response in a subject.

In a fifth aspect, the present invention concerns a vaccine for use in prevention and/or treatment of COVID-19, the vaccine comprising the peptide, the composition, the polynucleotide, the vector, and/or the host cell as disclosed above and defined in the claims.

### Description of Drawings

**Figure 1****:** RRM cross-spectrum based on 27 human and non-human interacting spike protein fragments. A single prominent common characteristic frequency is identified at 0.2827±0.0009.
**Figure 2****:** RRM cross-spectrum based on 9 human interacting coronavirus Spike-S1 protein fragments. A single prominent common characteristic frequency is identified at 0.3145±0.0019.
**Figure 3****:** RRM cross-spectrum based on 9 human interacting coronavirus Spike-S1 protein fragments and 10 ACE2 receptor protein fragments. A single prominent common characteristic frequency is identified at 0.3145±0.0019.
**Figure 4****:** RRM cross-spectrum based on 9 human interacting coronavirus Spike-S1 protein fragments and 5 red blood cell (RBC) Band3 protein fragments. A single prominent common characteristic frequency is identified at 0.3145±0.0019.
**Figure 5****:** ELISA assay of relative binding of Spike S1 to ACE2, following CovA peptide incubation with SARS-CoV-2 Spike-S1-Biotin according to the SARS-CoV-2 Spike inhibitor Screening Assay Kit provided for in Example 6. All concentrations were measured in 8 replicates. On the figure, a value of 1 represents relative value of positive control (0 µg/mL of peptide concentration). Error bars are calculated as standard deviations.
Figure 6: Legend: / - empty well. 1 (in grey) - CPE was observed, i.e., virus was not inhibited by the peptide in this concentration. 0 (in white) - no CPE was observed, i.e., virus was inhibited by the peptide in this concentration.
Figure 7: Graphical representation of data generated in Example 8. The data shows a dose-dependent relation between the concentration of CovA peptide, and the percentage of CPE inhibition observed. CovA concentration ranges from 6.25 to 200 µg/mL.
Figure 8: Graphical representation of data generated in Example 8. The data shows a dose-dependent relation between the concentration of CovA peptide, and the percentage of CPE inhibition observed. CovA concentration ranges from 1.56 to 200 µg/mL.
Figure 9: Relation between the inverse expression fold value as calculated in Example 8, and concentration of the CovA peptide. CovA peptide concentration ranges from 25 to 200 µg/mL, and viral concentration ranges from 6.25 to 1.56 TCID50 units.
Figure 10: Partial virus inhibition observed by reduced CPE after incubation with 200 µg/mL CovA peptide.
Figure 11: Cell culture control showing intact Vero E6 cells monolayer in 2% DMSO medium.
Figure 12: CovA peptide control sample (200 µg/mL concentration). No CPE is visible.
Figure 13: Virus back titration control. CPE caused by replication of SARS-CoV-2 in Vero E6 cells clearly observable.

### Detailed description

The spike glycoprotein of coronaviruses is the first part of the virus particle which attach to a host cell to initiate the infection. Consequently, disruption of this mechanism could be the most relevant target for design of treatments. To be able to design a universal treatment for all coronaviruses, common characteristic parameters for spike proteins from different strains should be identified and then use these parameters for further drug design. For that purpose, the inventors of the present application have utilised the Resonant Recognition Model (RRM), which proposes that protein biological function is characterised by certain periodicities or frequencies, within the distribution of free electron energies along a given protein. The RRM model is capable of identifying common characteristics for proteins having the same biological function and/or interaction, but not necessarily a high degree of homology.

### Biological activity

The novel peptides of the present invention are capable of modulating and/or inhibiting the ACE2/Spike-S1 interaction associated with SARS-CoV-2 viral infection in humans. The present inventors have achieved this effect by designing peptides to have a characteristic frequency which matches the characteristic frequency of the spike S1 protein of coronaviruses found to cause infections in humans.

In one embodiment, the peptide, the composition, the polynucleotide, the vector, or the host cell, of the invention is for use as a medicament.

In one embodiment, the peptide, the composition, the polynucleotide, the vector, or the host cell, of the invention is for use in prevention and/or treatment of a SARS-CoV-2 related disease in a subject.

In one embodiment, the disease treated herein is COVID-19.

In one embodiment, the peptide, the composition, the polynucleotide, the vector, or the host cell, of the invention is for inducing an immune response in a subject.

In one embodiment, the immune response is a mucosal immune response.

In one embodiment, the mucosal immune response is against SARS-CoV-2.

In one embodiment, the present disclosure concerns a vaccine for use in prevention and/or treatment of COVID-19, the vaccine comprising the peptide, the composition, the polynucleotide, the vector, or the host cell, of the invention and as defined in the claims.

In one embodiment, the peptide, the composition, the polynucleotide, the vector, or the host cell, as defined herein is capable of reducing the relative binding of spike S1 protein to ACE2 by 10%, such as by 20%, such as by 30%, such as by 40%, such as by 50%, such as by 60%, such as by 70%, such as by 80%, compared to a control receiving no treatment.

In one embodiment, the peptide, the composition, the polynucleotide, the vector, or the host cell, as defined herein is useful for treatment and/or prevention and/or amelioration of COVID-19 by prevention of CPE.

In one embodiment, the usefulness of the peptide in treatment and/or prevention and/or amelioration of COVID-19 is evaluated on the observation of cytopathic effect (CPE) or lack thereof, such as structural changes in a host cell resulting from viral infection. CPE occurs when the infecting virus causes lysis (dissolution) of the host cell or when the cell dies without lysis because of its inability to reproduce.

### Resonant Recognition Model (RRM)

All proteins can be considered as a linear sequence of their constitutive elements, i.e., amino acids and biological function of proteins is determined primarily by this linear sequence.

The RRM (Cosic, 1994 and Cosic, 1997) interprets this linear information by transforming a protein sequence into a numerical series and then into the frequency domain using digital signal processing methods Fast Fourier Transform (FFT).

Protein primary structure can be presented as a numerical series by assigning the relevant physical parameter value to each amino acid. It has been shown that the best correlation can be achieved with parameters which are related to the energy of delocalised electrons of each amino acid (calculated as Electron Ion Interaction Potential (EIIP), as presented in Table 1), (Cosic, 1994; Cosic, 1997 and Pirogova at al, 2003). The resulting numerical series represents the distribution of the free electron's energies along the protein molecule.

Protein numerical series are converted into spectra using FFT, pertaining the same information as original sequence.

By compering these protein spectra using the multiple cross-spectral function, it has been found that proteins with the same biological function and/or interaction have the same frequency in common which is then characterising protein biological function/interaction and is called characteristic frequency (Cosic, 1994; Cosic, 1997; Cosic at al, 2016 and Cosic at al, 2019).

The RRM technique revealed that proteins and their interacting targets share the same matching characteristic frequency with opposite phase at this frequency (Cosic, 1994; Cosic, 1997; Cosic at al, 2016 and Cosic at al, 2019).

### Peptide Design

Once the characteristic biological function of the protein is identified, it is possible to design new peptides with desired frequency components and consequently with desired biological functions (Cosic, 1994; Cosic, 1997; Cosic at al, 1994; Krsmanovic at al, 1998; Hearn at al, 2001; Achour at al, 2007; Almansour at al, 2012 and Istivan at al, 2011). The process of bioactive peptides design is as follows:
1. Determination of RRM characteristic frequency using multiple cross-spectral function for a group of protein sequences that share common biological function (interaction).
2. Determination of phases for the characteristic frequencies of a particular protein which is selected as the parent for agonist/antagonist peptide.
3. Calculation using Inverse Fourier Transform of the signal with characteristic frequency and phase. The minimal length of the designed peptide is defined by the characteristic frequency f as 1/f.
4. Determination of resulting amino acid sequence using tabulated EIIP parameter values from Table 1.

It should be stressed that the peptides determined by the RRM technique are artificial peptides, the sequences of which have been designed to have only desired RRM frequency and phase, consequently expressing desired biological function/interaction.

This approach has been already successfully applied and experimentally tested in design of FGF (Cosic at al, 1994), HIV envelope protein analogue (Krsmanovic at al, 1998; Hearn at al, 2002 and Achour at al, 2007) and peptide to mimic myxoma virus oncolytic function (Almansour at al, 2012 and Istivan at al, 2011).

The present invention makes use of the above RRM procedure for design of peptides capable to block SARS-CoV-2 spike proteins interaction with human ACE2 receptor.

**Table 1 - list of calculated EIIP values for all 20 natural amino acids**

| Amino Acids | PEII (Ry*) |
|---|---|
| Leu (L) | 0.0000 |
| Ile (I) | 0.0000 |
| Asn (N) | 0.0036 |
| Gly (G) | 0.0050 |
| Val (V) | 0.0057 |
| Glu (E) | 0.0058 |
| Pro (P) | 0.0198 |
| His (H) | 0.0242 |
| Lys (K) | 0.0371 |
| Ala (a) | 0.0373 |
| Tyr (Y) | 0.0516 |
| Trp (W) | 0.0548 |
| Gln (Q) | 0.0761 |
| Met (M) | 0.0823 |
| Ser (S) | 0.0829 |
| Cys (C) | 0.0829 |
| Thr (T) | 0.0941 |
| Phe (F) | 0.0946 |
| Arg (R) | 0.0959 |
| Asp (D) | 0.1263 |

| | |
|---|---|
| * Ry = Rydberg unit | |

In one embodiment, the peptides of the invention are characterized by frequency components that match the S1/ACE2 interaction compatible with SARS-CoV-2 infections.

In one embodiment, the one or more characteristic frequency component is selected from f1 and f2.

In one embodiment, the one or more characteristic frequency component f1 is 0.2827±0.0009, and f2 is 0.3145±0.0019.

Retro-peptides are peptides consisting of the same sequence of amino acids, but in reverse order. Inverso-peptides are peptides consisting of the same sequence but composed of D-amino acids. Retro-inverso peptides are thus peptides whose amino acid sequence is reversed and the α-center chirality of the amino acid subunits is inverted as well. Usually, these types of peptides are designed by including D-amino acids in the reverse sequence to help maintain side chain topology similar to that of the original L-amino acid peptide and make them more resistant to proteolytic degradation. Other reported synonyms for these peptides in the scientific literature are: Retro-inverse Peptides, All-D-Retro Peptides, Retro-Enantio Peptides, Retro-Inverso Analogs, Retro-Inverso Analogues, Retro-Inverso Derivatives, and Retro-Inverso Isomers.

In one embodiment, the peptides of the present invention are formulated as peptidomimetics, such as retro-peptides, inverso-peptides, and retro-inverso peptides.

In one embodiment, the peptides of the present invention may comprise any range from all L-amino acids to all D-amino acids.

Thus, the present disclosure provides in one embodiment a peptide, wherein the peptide comprises an amino acid sequence selected from the group consisting of SEQ ID NO: 1, SEQ ID NO: 2, SEQ ID NO: 3, SEQ ID NO: 4, SEQ ID NO: 5, and SEQ ID NO: 6, or a sequence variant thereof differing by one or two amino acid residues from said SEQ ID NO.

In one embodiment, the sequence variant differs by one amino acid.

In one embodiment, the differing one or two amino acids of the sequence variant are generated by substitution, addition and/or deletion.

Sequence variants may differ by one or more conservative and/or semi-conservative amino acid substitutions, which typically have minimal influence on the secondary and tertiary structure and hydrophobic/hydrophilic nature of a protein and/or peptide. Sequence variants may also differ by one or more non-conservative amino acid substitutions, deletions, or insertions, which do not abolish the native biological activity.

In one embodiment, the sequence variant comprises one or more of conservative, semi-conservative, and non-conservative substitutions.

In one embodiment, the peptides of the present invention comprise at least 16 amino acid residues, such as at least 17 amino acid residues, such as at least 18 amino acid residues, such as at least 20 amino acid residues, such as at least 21 amino acid residues, such as at least 22 amino acid residues, such as at least 30 amino acid residues, such as at least 50 amino acid residues.

In one embodiment, the peptides of the present invention comprise no more than 50 amino acid residues, such as no more than 30 amino acid residues, such as no more than 22 amino acid residues, such as no more than 21 amino acid residues, such as no more than 20 amino acid residues, such as no more than 19 amino acid residues, such as no more than 18 amino acid residues.

In one embodiment, the peptides of the present invention comprise in the range of 16 to 50 amino acid residues, such as in the range of 16 to 30 amino acid residues, such as in the range of 16 to 22 amino acid residues, such as in the range of 16 to 20 amino acid residues, such as in the range of 17 to 19 amino acid residues.

In one embodiment, the peptides of the present invention are capable of binding to the SARS-CoV-2 Spike S1 protein.

In one embodiment, the peptides of the present invention are capable of inhibiting and/or blocking the binding of the Spike-S1 protein of SARS-CoV-2 to the human ACE2 receptor.

In one embodiment, the peptides of the present invention are further conjugated to a moiety.

In one embodiment, the moiety is selected from the group consisting of PEG, monosaccharides, fluorophores, chromophores, and radioactive compounds.

In one embodiment, the moiety is a detectable moiety.

In one embodiment, the peptides of the present invention are further modified by glycosylation, phosphorylation, PEGylation, amidation, esterification, acylation, acetylation and/or alkylation.

In one embodiment, the peptides of the present invention are further modified by alkylation, such as C₁₋₆ alkylated.

In one embodiment, the peptides of the present invention comprise either proteinogenic or non-proteinogenic amino acids.

In one embodiment, the peptides of the present invention comprise SEQ ID NO: 1 or a sequence variant thereof differing by one or two amino acid residues.

In one embodiment of the present disclosure, the peptides comprise or consist of the amino acid sequence HRWKQWWWAQCPYDKLDM (SEQ ID NO: 1)

In one embodiment of the present disclosure, the peptide comprises or consists of the amino acid sequence LWDKERTNWDAERRGYDY (SEQ ID NO: 2)

In one embodiment of the present disclosure, the peptide comprises or consists of the amino acid sequence QDEWDHKDAPDWGRCLTR (SEQ ID NO: 3)

In one embodiment of the present disclosure, the peptide comprises or consists of the amino acid sequence RHWFWWQQCWARMVTDLY (SEQ ID NO: 4)

In one embodiment of the present disclosure, the peptide comprises or consists of the amino acid sequence DWNTDPKDQICDPHDQLQ (SEQ ID NO: 5)

In one embodiment of the present disclosure, the peptide comprises or consists of the amino acid sequence YEDQNRTLCRVWDPKDKP (SEQ ID NO: 6)

One embodiment of the present disclosure is a polynucleotide encoding the peptide as defined herein.

One embodiment of the present disclosure is a vector comprising a polynucleotide encoding the peptide as defined herein.

One embodiment of the present disclosure is a host cell comprising a polynucleotide, or a vector comprising a polynucleotide, encoding the peptide as defined herein.

In one embodiment, the host cell is selected from the group consisting of bacterial cell, mammalian cell, and human cell.

### Subject

The present composition comprising the peptides of the invention is for use in the treatment of viral diseases or disorders as described herein in a subject in need thereof.

In one embodiment, the subject is infected with a coronavirus. In another embodiment, the treatment is prophylactic and administered to a subject in risk of being infected with SARS-CoV-2.

In one embodiment, the subject is infected with SARS-CoV-2

In one embodiment the subject is a mammal. In particular the subject may be a human.

In other embodiments the subject is an animal, such as a pet, including for example a cat, a dog, a rabbit, a horse, or a farm animal such as cattle or poultry, for example a cow, a bull, a sheep, a goat, a mink, a pig, a chicken, or a turkey.

In one embodiment, subjects are elderly above 65 years of age.

In one embodiment, elderly subjects are 70 years of age or above.

### Combination therapies

In one embodiment, the composition comprising the peptides of the invention, or a pharmaceutically acceptable derivative thereof as disclosed herein, comprises, separately or together, one or more additional active pharmaceutical ingredients.

In one embodiment, said one or more additional active pharmaceutical ingredients are used for the treatment of a viral disease or disorder.

In one embodiment, said one or more additional active pharmaceutical ingredients are used for the treatment of symptomatic COVID-19.

In one embodiment, the composition comprising the peptides of the invention, or a pharmaceutically acceptable derivative thereof for use as disclosed herein is an add-on therapy to existing therapies.

In one embodiment, the composition comprising the peptides of the invention, or a pharmaceutically acceptable derivative thereof for use as disclosed herein is an add-on therapy to one or more additional therapies used for the treatment of viral diseases or disorders.

In one embodiment, said add-on therapy includes one or more of oxygen on nasal catheter and mechanical ventilation.

### Routes of administration

It will be appreciated that the preferred route of administration will depend on the general condition and age of the subject to be treated, the nature of the condition to be treated, the location of the tissue to be treated in the body and the active ingredient chosen.

In one embodiment, the administration is by systemic administration, local administration, enteral administration, or parenteral administration. Appropriate dosage forms for such administration may be prepared by conventional techniques.

### Systemic administration

Systemic administration is capable of introducing the compound into the blood stream to ultimately target the sites of desired action.

Such routes of administration are any suitable routes, such as an enteral route, the oral, rectal, nasal, pulmonary, buccal, sublingual, transdermal, intracisternal, intraperitoneal, and parenteral (including subcutaneous, intramuscular, intrathecal, intravenous, and intradermal) route.

In one embodiment, the administration route is intravenous, oral, mucosal, or sublingual.

### Oral administration

Oral administration is normally for enteral drug delivery, wherein the compound is delivered through the enteral mucosa. Syrups and solid oral dosage forms, such as tablets, capsules, and the like, are commonly used.

In one embodiment, the composition comprising any one of SEQ ID NOs: 1 to 6 is administered by oral administration.

### Parenteral administration

Parenteral administration is any administration route not being the oral/enteral route whereby the medicament avoids first-pass degradation in the liver. Accordingly, parenteral administration includes any injections and infusions, for example bolus injection or continuous infusion, such as intravenous administration, intramuscular administration, subcutaneous administration. Furthermore, parenteral administration includes inhalations and topical administration.

Accordingly, the compound may be administered topically to cross any mucosal membrane of an animal to which the biologically active substance is to be given, e.g. in the nose, vagina, eye, mouth, genital tract, lungs, gastrointestinal tract, or rectum, preferably the mucosa of the nose, or mouth, and accordingly, parenteral administration may also include buccal, sublingual, nasal, rectal, vaginal and intraperitoneal administration as well as pulmonal and bronchial administration by inhalation or installation. Also, the compound may be administered topically to cross the skin.

In one embodiment, the composition comprising any one of SEQ ID NOs: 1 to 6 is administered by parenteral administration

In one embodiment, the composition comprising any one of SEQ ID NOs: 1 to 6 is administered by topical administration

### Local treatment

The peptides or compositions as disclosed herein is in one embodiment used as a local treatment, i.e., is introduced directly to the site(s) of action. Accordingly, the peptide or composition may be applied to the skin or mucosa directly, or the peptide or composition may be injected into the site of action, for example into the diseased tissue or to an end artery leading directly to the diseased tissue.

### Dosage

According to the present disclosure, the composition comprising the peptides of the invention is administered to individuals in need of treatment in pharmaceutically effective doses. A therapeutically effective amount of a compound is an amount sufficient to cure, prevent, reduce the risk of, alleviate or partially arrest the clinical manifestations of a given disease and its complications. The amount that is effective for a particular therapeutic purpose will depend on the severity and the sort of the disorder as well as on the weight and general state of the subject. The compound may be administered one or several times per day, such as from 1 to 8 times per day, such as from 1 to 6 times per day, such as from 1 to 5 times per day, such as from 1 to 4 times per day, such as from 1 to 3 times per day, such as from 1 to 2 times per day, such as from 2 to 4 times per day, such as from 2 to 3 times per day. Alternatively, the compound may be administered less than once a day, for example once a day, such as once every second day, for example once every third day, such as once every fourth day, for example once every fifth day, such as once every sixth day, for example once every week.

In one embodiment the composition comprising the peptides of the invention is administered in a therapeutically effective amount, such as in an amount of 1 mg to 1000 mg of a peptide comprising any one of SEQ ID NOs: 1 to 6 per day.

It follows that in one embodiment the composition comprising the peptides of the invention is administered in an amount of 1 mg to 1000 mg, such as 1 to 200 mg, 200 to 400 mg, 400 to 600 mg, 600 to 800 mg, 800 to 1000 mg, for example 500 to 1000 mg per day.

Per day means the dosage may be given in one dosage or divided in multiple dosages per day, including once a day (QD), twice a day (BID) and/or three times a day (TID).

In one embodiment the composition comprising the peptides of the invention is administered in an amount of 100 mg once daily, 200 mg once daily, 300 mg once daily, 400 mg once daily, 500 mg once daily, 600 mg once daily, 700 mg once daily, 800 mg once daily, 900 mg once daily or 1000 mg once daily.

In another embodiment the composition comprising the peptides of the invention is administered in an amount of 0.01 mg/kg bodyweight to 40 mg/ kg bodyweight, such as 0.01 mg/ kg bodyweight to 0.5 mg/ kg bodyweight, 0.5 mg to 5 mg/ kg bodyweight, 5 to 20 mg/ kg bodyweight, 20 to 40 mg/ kg bodyweight.

### Pharmaceutical composition

Whilst it is possible for the peptides of the present invention to be administered as the raw peptide, it is preferred to present them in the form of a pharmaceutical formulation. Accordingly, the present invention further provides a pharmaceutical formulation, which comprises the peptides of the present invention or a pharmaceutically acceptable salt thereof, and a pharmaceutically acceptable carrier, therefore. The pharmaceutical formulations may be prepared by conventional techniques, e.g., as described in Remington: The Science and Practice of Pharmacy 2005, Lippincott, Williams & Wilkins.

One embodiment of the present disclosure is a composition comprising the peptides of the invention.

In one embodiment the composition comprising the peptides of the invention is a pharmaceutical composition, such as a pharmaceutically safe composition.

### Formulation

The composition comprising the peptides of the invention may be administered in any suitable way e.g., orally, sublingually, or parenterally, and it may be presented in any suitable form for such administration, e.g., in the form of solutions, suspension, aerosols, tablets, capsules, powders, syrups, implant or dispersions for injection.

In one embodiment, the composition comprising the peptides of the invention is formulated as a suspension.

In one embodiment, the composition comprising the peptides of the invention is formulated as an aerosol.

In one embodiment, the composition comprising the peptides of the invention is formulated as an oral dose form, such as a solid oral dose form or pharmaceutical entity, such as tablets or capsules, or a liquid oral dose form. Methods for the preparation of solid pharmaceutical preparations are well known in the art.

In another embodiment the composition comprising the peptides of the invention is formulated as an injectable dose form.

The composition comprising the peptides of the invention may be administered alone or in combination with pharmaceutically acceptable carriers or excipients, in either single or multiple doses. The pharmaceutical compositions may be formulated with pharmaceutically acceptable carriers or diluents as well as any other known adjuvants and excipients in accordance with conventional techniques such as those disclosed in Remington: The Science and Practice of Pharmacy, 19 Edition, Gennaro, Ed., Mack Publishing Co., Easton, Pa., 1995.

In one embodiment, the composition comprising the peptides of the invention comprises one or more independently selected from each of adjuvants, excipients, carriers, and nutrients.

### Examples

### Example 1 - determination of characteristic frequency (frequencies) for viral spike proteins

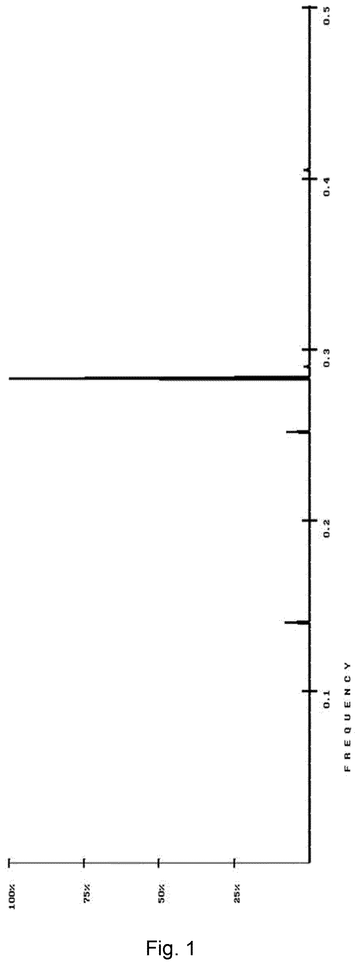

Application of RRM to the analysis of viral spike proteins. The algorithm was applied to a total of 27 viral spike proteins. The analysis procedure comprises the following steps:
1. each amino acid sequence was converted to the related numerical series by representing each amino acid with the corresponding value of the electron ion interaction potential (ElIP);
2. this numerical series was converted into a numerical spectrum using Fast Fourier Transform (FFT);
3. spectra were mutually compared using cross-spectral analysis with the aim to extract common frequency components;
4. multiple cross-spectral analysis was then applied over each group of the protein sequences (27 viral spike proteins) to extract common characteristic frequency components for the viral spike protein interaction. In the present example, a characteristic frequency f1=0.2827±0.0009 was extracted (Figure 1).

With the characteristic RRM frequency identified for 27 spike proteins, it was possible, using inverse procedure, to design a variety of analogous peptides which have the same desired spectral characteristics, and therefore expected to have desired biological function. These peptides were designed *de novo.* With the designed peptides, rigorous criteria were employed to ensure that each de novo peptide had desired RRM frequency and phase. The procedures and criteria for the design of these peptides involved the following steps:
5. determination of the characteristic frequencies for the analysed group of viral spike proteins as described above in 1-4;
6. definition of the characteristic phases at the characteristic frequencies for the viral spike proteins, chosen as target for the peptide design;
7. derivation of a new numerical sequence from the knowledge of these characteristic frequencies and phases using inverse discrete Fourier transform procedures;
8. determination of the amino acid corresponding to each element of this new numerical sequence from values of EIIP (Table 1).

The peptides obtained in this way have the common spectral characteristics with the 27 viral spike proteins used in the basis set and can be proposed to exhibit a same function/interaction properties, although they do not have any sequence similarity with any of these viral spike proteins.

### Example 2 - determination of characteristic frequency (frequencies) for human interacting coronavirus Spike S1 protein fragments

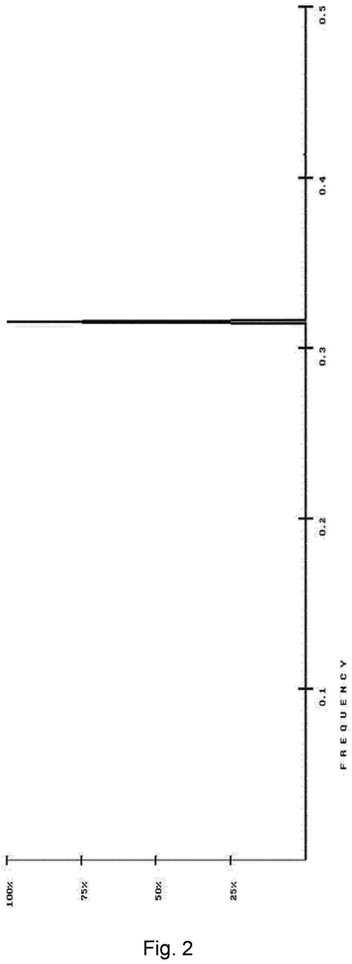

Applying the procedure as set out in Example 1, multiple cross-spectral analysis was then applied over each group of the protein sequences (9 human interacting coronavirus spike S1 protein fragments) to extract common characteristic frequency components for the interaction. In the present example, a characteristic frequency f2=0.3145±0.0019 was extracted (Figure 2).

### Example 3 - determination of characteristic frequency (frequencies) for combination of human interacting coronavirus Spike S1 protein fragments and ACE2 receptors.

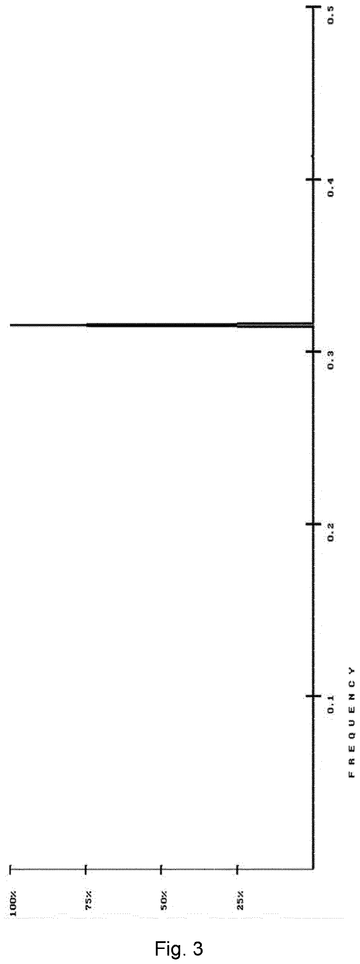

Applying the procedure as set out in Example 1, multiple cross-spectral analysis was then applied over each group of the protein sequences (9 human interacting coronavirus spike S1 protein fragments and 10 ACE2 receptor proteins) to extract common characteristic frequency components for the interaction. In the present example, a characteristic frequency f2=0.3145±0.0019 was extracted, which is identical to that obtained in Example 2 using only the coronavirus spike S1 protein fragment as basis (Figure 3).

### Example 4 - determination of characteristic frequency (frequencies) for 9 human interacting coronavirus Spike S1 protein fragments and RBC Band3 proteins

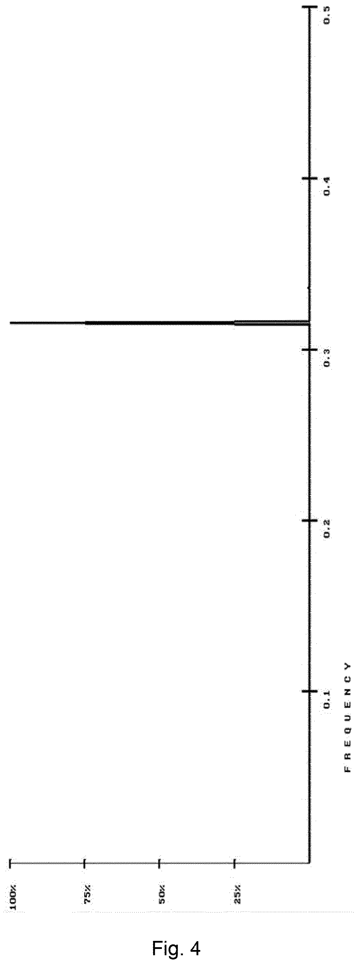

Applying the procedure as set out in Example 1, multiple cross-spectral analysis was then applied over each group of the protein sequences (9 human interacting coronavirus spike S1 protein fragments and 5 RBC Band3 proteins) to extract common characteristic frequency components for the interaction. In the present example, a characteristic frequency f2=0.3145±0.0019 was extracted, which surprisingly is identical to that obtained in Example 2 using only the coronavirus spike S1 protein fragments as basis (Figure 4).

### Example 5 - peptide preparation

Based on the characteristic frequencies extracted in Examples 1-4, six (6) custom peptides were designed having either one or both of characteristic frequencies f1 and f2, with the phases being identical or opposite.

| Peptide | F1 = 0.2827 | Phase 1 = +2.77rad | F2 = 0.3145 | Phase 2 = -1.74rad |
|---|---|---|---|---|
| CovA | X | Same | X | Same |
| CovB | X | Same | - | - |
| CovC | - | - | X | Same |
| CovD | X | Opposite | X | Opposite |
| CovE | X | Opposite | - | - |
| CovF | - | - | X | Opposite |

Peptide CovA having both frequencies f1 and f2 with the same phases as virus spike proteins.

Peptide CovB having only frequency f1 with the same phase as virus spike protein.

Peptide CovC having only frequency f2 with the same phase as virus S1 spike protein.

Peptide CovD having both frequencies f1 and f2 with the opposite phases as virus spike proteins.

Peptide CovE having only frequency f1 with the opposite phase as virus spike protein.

Peptide CovF having only frequency f2 with the opposite phase as virus S1 spike protein.

Peptides were provided by a third party manufacturer and made to order. The ordered peptides were supplied as lyophilized powder and stored at room temperature before use. All peptides were at least 95% purity as evidenced by HPLC and MS analysis.

Physico-chemical properties of each provided peptide were pre-estimated using the peptide property calculator INNOVAGEN (presently available at www.pepcalc.com). Peptide solubility was checked with NanoDrop 1000 (ThermoFischer Scientific, US).

In case of peptides with good water solubility, 1 mg of each lyophilized peptide was dissolved in 5 mL phosphate-buffered saline (PBS) pH = 7.4. Stock solution was prepared with a peptide concentration of 200 µg/mL and stored at - 20°C until further use. Two-fold serial dilutions were made in PBS up to 1:512 to prepare peptide concentrations for the experiment in the range of 0.39 to 200 µg/mL.

In case of peptides with poor water solubility, 2.5 mg of each lyophilized peptide was dissolved in 500 µL of 100% dimethylsulfoxide (DMSO) to have a stock solution in the concentration of 5.0 mg/mL and solutions were stored at - 20°C. Before the experiments, the stock solution was diluted 25-fold with MilliQ water to obtain the highest working concentration of the peptide solution 200 µg/mL in 4% DMSO. Solutions were stored at - 20°C. Further two-fold serial dilutions were made in 4% DMSO up to 1:512 to prepare peptide concentrations for the experiment in the range of 0.39 to 200 µg/mL.

The effect of all peptide concentrations on the SARS-CoV-2 Spike S1 and ACE2 interaction was tested in 8 replicates.

### Example 6 - in vitro inhibition of S1/ACE2 assay

The following is based on the CovA peptide comprising SEQ ID NO: 1. The procedure is however general for all peptides of the present disclosure.

To test the blocking of the SARS-CoV-2 Spike S1 / ACE2 interaction, the peptides of the invention were incubated with either ACE2-His or SARS-CoV-2 Spike-S1-Biotin using either the ACE2 Inhibitor Screening Assay Kit or the SARS-CoV-2 Spike inhibitor Screening Assay Kit as procured from BPS Bioscience, San Diego, CA, US. For both assays, the tests were made according to the manufacturer's instructions. Prior to the test, peptides were thawed at room temperature for 30 minutes before being kept in a thermoblock at 37°C for an additional 30 minutes.

The employed assays are ELISA assays, and the read-out is evaluated based on the chemiluminescence as measured using a Citation 3 Imaging Reader (BioTek, US).

### Results

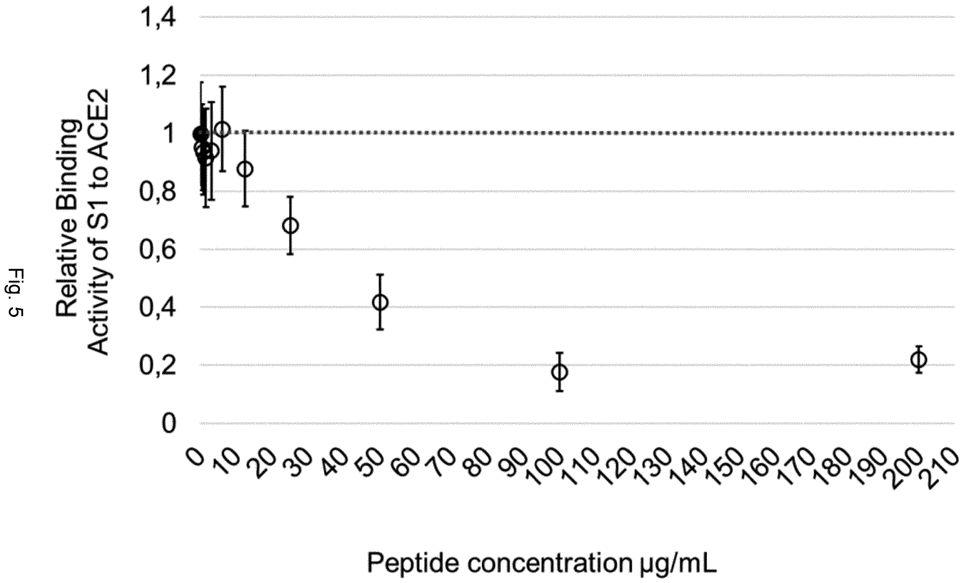

The results representing the effect of blocking the SARS-CoV-2 Spike S1 - ACE2 interaction using the CovA peptide are shown in Figure 5. A value of 1 represents the relative value of a positive control (0 µg/mL of peptide concentration), which means the highest binding activity between native SARS-CoV-2 Spike S1 and native ACE2. If the addition of peptides of the invention induces deviation from the value 1 to lower values which are significantly different than the value obtained by the positive control, it means that the tested peptide has an inhibitory effect on the SARS-CoV-2 Spike S1 -ACE2 interaction.

### Conclusion

The presented data clearly shows a statistically significant reduction in the relative binding activity between Spike S1 of SARS-CoV-2 and ACE2. Specifically, when applying the highest effective peptide concentration of 200 µg/mL, 80% of the binding between Spike S1 of SARS-CoV-2 and ACE2 is prevented, compared to baseline (0 µg/mL).

### Example 7 - screening tolerability of CovA peptide

### Materials and Methods

Serial twofold dilutions of the tested peptide were prepared in cell culture medium containing 4% or 2% DMSO. The working dilution of SARS-CoV-2 virus (100 TCID50 units) was prepared in cell culture medium containing 4% or 2% DMSO. An equal volume (25 µl) of serial twofold dilutions of peptide and working dilution of SARS-CoV-2 were mixed in a cell culture plate and incubated at 37° C for an hour.

After the incubation, 50 µl of 3 × 10⁵ Vero E6 cells/ml were added to each well. To ensure optimal results and to determine that the adequate virus TCID50 units were used in the test, virus was back titrated and reference positive serum sample, peptide CovA control and cell control were included in each plate. The plates were incubated at 37° C with 5% CO₂ atmosphere for 5 days and the results of the inhibition/neutralisation were evaluated based on cytopathic effect (CPE)

To verify the tolerability of Vero E6 cells towards DMSO, a screening of 1%, 2%, and 4% of DMSO in cell culture medium was also evaluated. It was determined that the concentration of 2% or less of DMSO was tolerable by the cell line. Thus, all CovA peptide assays were prepared in 2% DMSO.

### Results

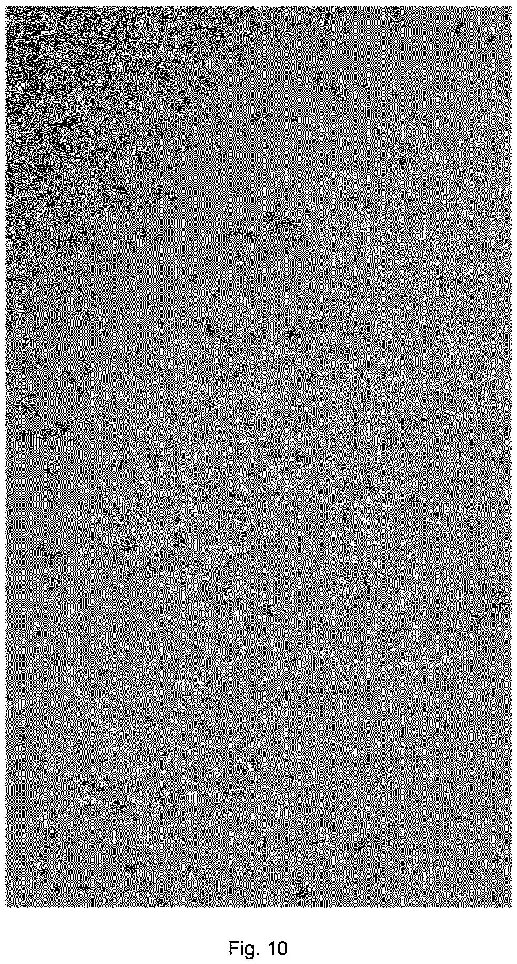

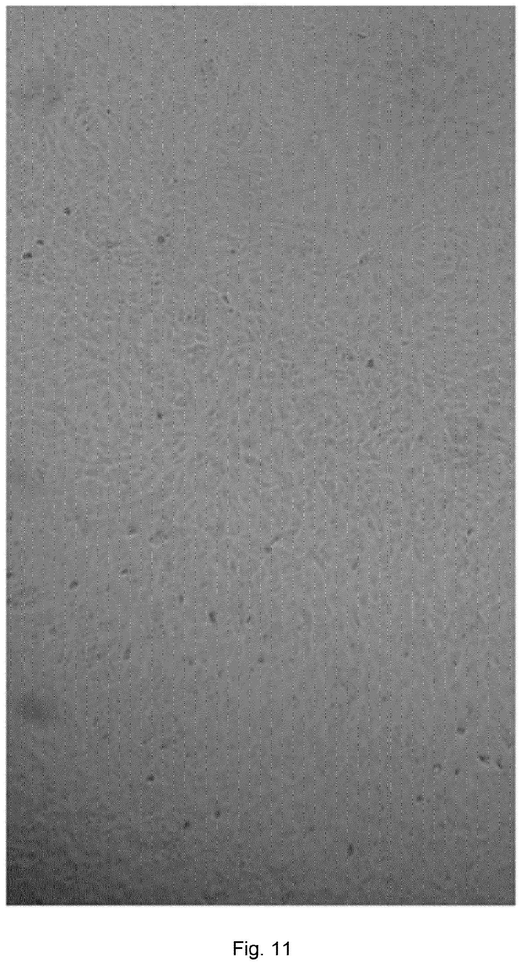

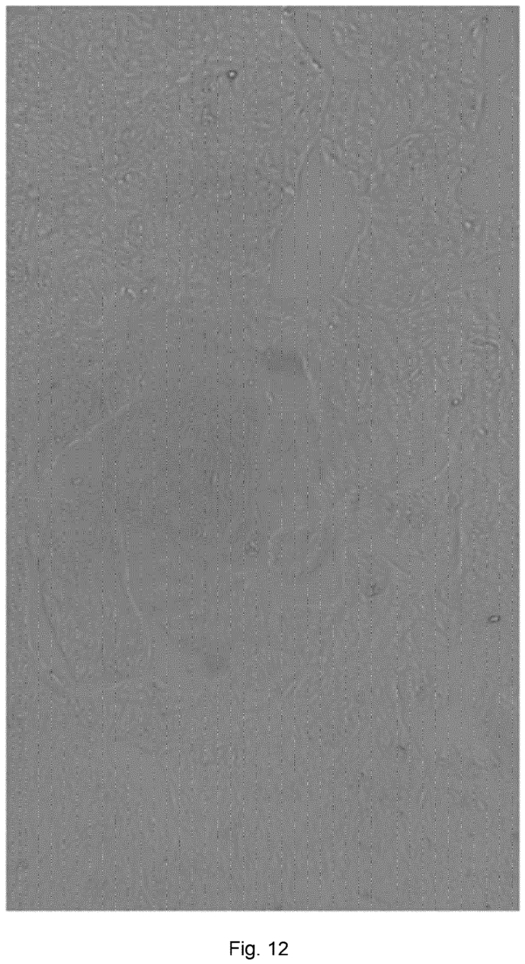

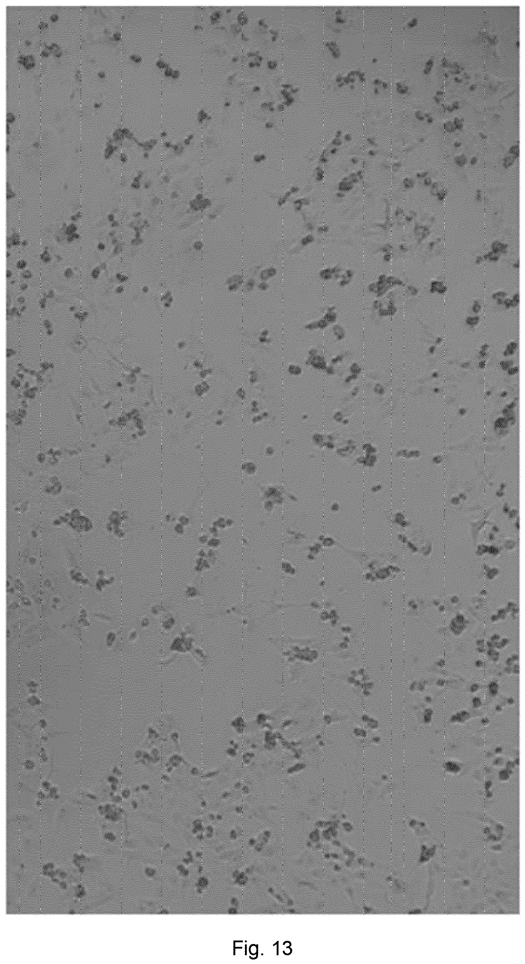

The efficacy of CovA peptide towards SARS-CoV-2 was screened at two concentrations, 25 µg/mL and 200 µg/mL. Both concentrations demonstrated good tolerability as evaluated based on lack of CPE in both the cell culture control sample (200 µg/mL, Figure 11) comprising 2% DMSO, and the CovA control sample (200 µg/mL, Figure 12), also comprising 2% DMSO. In the samples where the CovA peptide was incubated with SARS-CoV-2 virus, partial inhibition was observed, as a reduced CPE (200 µg/mL, Figure 10), compared to the virus back titration control sample (200 µg/mL, Figure 13).

### Conclusion

In summary, the presented results show that the CovA peptide is well tolerated in cell culture medium comprising up to 2% DMSO. Additionally, incubation of CovA with SARS-CoV-2 virus drastically reduces the observed CPE as evidenced in Figure 10, compared to the virus back titration control sample containing no CovA peptide (Figure 13).

### Example 8 - screening virus inhibition of CovA peptide

### Materials and Methods

Peptide concentration: based on the obtained results from previous examples, the maximal chosen concentration of the peptide was 200 µg/mL and peptide were serially diluted in two-fold dilutions up to 1:128. Serial two-fold dilutions of the tested peptide were prepared in cell culture medium containing 2 % DMSO.

Virus concentration: A concentration of 50 TCID50 units was chosen as maximum virus concentration that was used in the tests. Serial two-fold dilutions of the virus up to 1:128 were prepared in cell culture medium.

Controls included in the tests: the reference positive sera were diluted in the same dilutions as tested peptide and then tested with the same virus concentrations, the virus titration control, peptide control and cell culture control were also included.

Protocol: Optimisation of the peptide - virus concentration was performed in quadruplicate (each peptide dilution per each virus dilution in 4 wells of the 96-well microtiter plate), so the interpretation of the results based on four replicates were possible. The results were determined on the observed cytopathic effect (cpe). An equal volume (25 µl) of serial twofold dilutions of peptide and serial twofold dilutions of SARS-CoV-2 were mixed in a cell culture plate and incubated at 37° C for an hour. After the incubation, 50 µl of 3 × 10⁵ Vero E6 cells/ml were added to each well. The plates were incubated at 37° C with 5% CO₂ atmosphere for 5 days and the results of the inhibition/neutralisation were evaluated based on cytopathic effect (CPE).

Detection of viral RNA by real-time PCR and quantification of viral load by calculation of the ΔCt values: The results are given in the Ct values. Ct value indicated threshold cycle which reflects cycle number at which the fluorescence generated within a reaction crosses the threshold (positive-negative cut-off value). It is inversely correlated to the logarithm of the initial copy number of RNA. To calculate expression fold value of the virus growth, the ΔCt was used. ΔCt means the difference between the Ct value of a sample obtained after incubation and the Ct for the sample obtained before incubation in case that in both occasion RNA was extracted from the equal volume of the sample. ΔCt was used to calculate expression fold value by the equation: expression fold value = 2^{-ΔCt}.

### Results

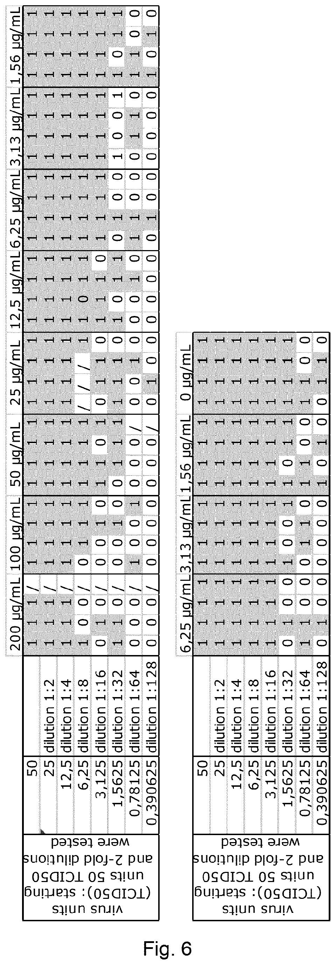

The results of the optimisation of the peptide-to-virus concentration performed in quadruplicate (each peptide dilution per each virus dilution in 4 wells of the 96-well microtiter plate) are given in Figure 6. The plates were incubated for 5 days and the results of the inhibition/neutralisation were evaluated based on cytopathic effect (cpe). A blank control containing no added CovA peptide is also provided for in Figure 6.

As evidenced from the control, viral dilutions of 1:64 and 1:128 were only found together with CPE in 50% of the tested wells.

Based on real-time PCR, Ct values were measured and averaged over a sample size as presented in Table 2.

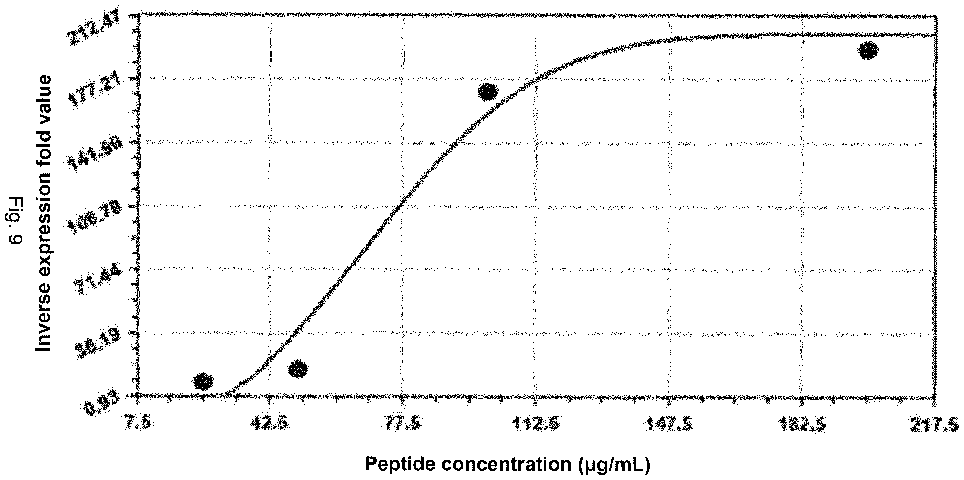

**Table 2 - relation between expression fold value given as 2^{-ΔCt} and peptide concentration (data plotted in Figure 9).**

| Peptide concentration (µg/ml) | Ct (average) | ΔCt | 2^{-ΔCt} | 1/(2^{-ΔCt}) |
|---|---|---|---|---|
| 0 | 14,06 | 0 | 1 | 1 |
| 25 | 17,29 | 3,23 | 0,1066 | 9,3 |
| 50 | 18,05 | 3,99 | 0,0629 | 15,180 |
| 100 | 21,47 | 7,41 | 0,0059 | 170,1 |
| 200 | 21,66 | 7,6 | 0,0051 | 194 |

### Conclusion

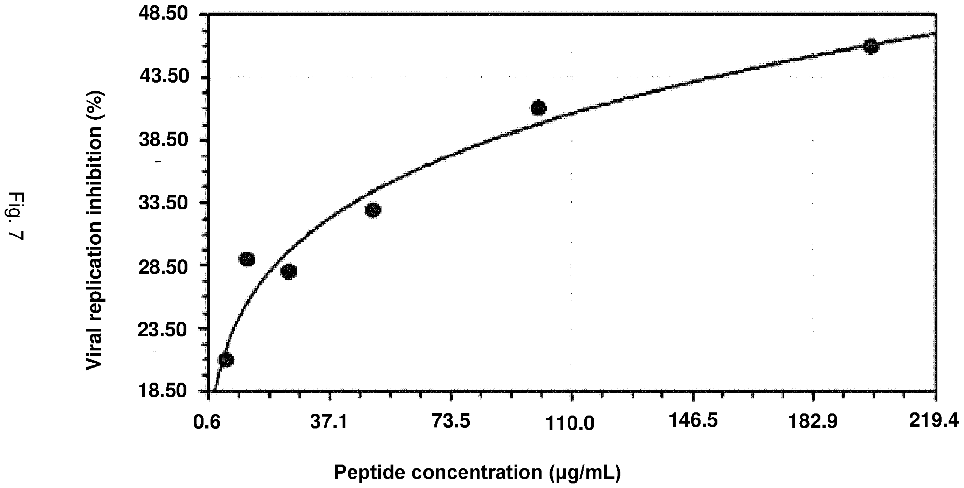

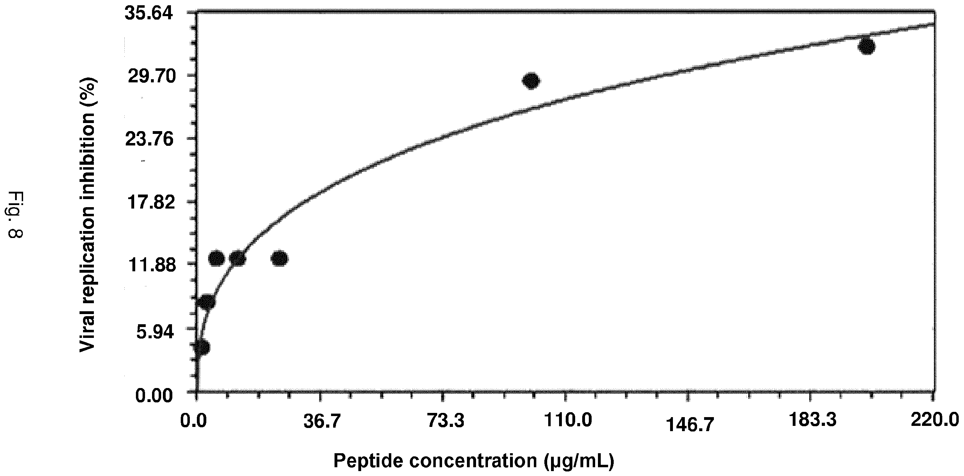

Although there is some variability that influence the curve (seen in Figure 7), peptide CovA inhibits the SARS-CoV-2 in a concentration dependant manner. As it can be seen from the curve, the viral replication was inhibited in the highest number (46 %) of wells at the highest (200 ug/ml) concentration of the peptide and the lowest (21 %) at the 6.25 ug/ml concentration. Lower peptide concentrations (3.125 and 1.5625 ug/ml) were omitted from the present analysis but included in Figure 8 for completeness.

The results of ΔCt and expression fold values shoved that the expression of viral RNA inversely correlates with the concentration of the peptide, i.e., the higher concentration of peptide, the lower expression of viral RNA. Based on the results, it could be estimated that the expression of viral RNA was 194-fold lower in the presence of CovA in 200 µg/ml concentration compared to a control comprising only SARS-CoV-2 and Vero E6 cells.

### Sequence overview

A summary of peptide sequences disclosed in the present application is presented here below:

| SEQ ID NO | Sequence | Comment |
|---|---|---|
| 1 | HRWKQWWWAQCPYDKLDM | CovA |
| 2 | LWDKERTNWDAERRGYDY | CovB |
| 3 | QDEWDHKDAPDWGRCLTR | CovC |
| 4 | RHWFWWQQCWARMVTDLY | CovD |
| 5 | DWNTDPKDQICDPHDQLQ | CovE |
| 6 | YEDQNRTLCRVWDPKDKP | CovF |

### References

1. Cosic, I. (1994) Macromolecular bioactivity: is it resonant interaction between macromolecules?-Theory and applications. IEEE Trans. on Biomedical Engineering, 41, 1101-1114.
2. Cosic, I. (1997) The Resonant Recognition Model of Macromolecular Bioactivity: Theory and Applications. Birkhauser Verlag, Basel.
3. Pirogova, E. at al (2003) Investigation of the applicability of Dielectric Relaxation properties of amino acid solutions within the Resonant Recognition Model. IEEE Transactions on NanoBioscience, 2(2), 63-69.
4. Cosic, I. at al (2016) Analysis of Tumor Necrosis Factor Function Using the Resonant Recognition Model. Cell Biochemistry and Biophysics, 74(2), 175-180.
5. Cosic, I. at al (2019) Analysis of Protein-Receptor on an Example of Leptin-Leptin Receptor Interaction Using the Resonant Recognition Model. MDPI Appl. Sci., 9, 5169.
6. Cosic, I. at al (1994) In vitro inhibition of the actions of basic FGF by novel 16 amino acid peptides. Molecular and Cellular Biochemistry, 130, 1-9.
7. Krsmanovic, V. at al (1998) Investigation Into the Cross-reactivity of Rabbit Antibodies Raised against Nonhomologous Pairs of Synthetic Peptides Derived from HIV-1 gp120 proteins. J.Peptide Res, 52(5), 410-412.
8. Hearn, M.T.W. at al (2001) Peptides Immunologically related to proteins expressed by a viral agent, having a sequence of amino acids ordered by means of protein informational method. US Patent 6, 294, 174.
9. Achour, A. at al (2007) Induction of Human Immunodeficiency Virus (HIV-1) Envelope Specific Cell-Mediated Immunity by a Non-Homologus Synthetic Peptide. PLoS ONE, 11, 1-12.
10. Almansour, N. at al (2012) Investigation of cytotoxicity of negative control peptides versus bioactive peptides on skin cancer and normal cells: a comparative study. Future Medicinal Chemistry, 4(12), 1553-1565.
11. Istivan, T. at al (2011) Biological effects of a De Novo designed myxoma virus peptide analogue: Evaluation of cytotoxicity on tumor cells. Public Library of Science (PLoS) ONE, 6(9), 1-10.

## Claims

1. A peptide, wherein the peptide comprises the amino acid sequence of SEQ ID NO: 1, or a sequence variant thereof differing by one or two amino acid residues from said SEQ ID NO.

2. The peptide according to any one of the preceding claims, wherein the one or two amino acids of the sequence variant are realized by substitution, addition and/or deletion.

3. The peptide according to any one of the preceding claims, wherein the peptide comprises in the range of 16 to 50 amino acid residues, such as in the range of 16 to 30 amino acid residues, such as in the range of 16 to 22 amino acid residues, such as in the range of 16 to 20 amino acid residues, such as in the range of 17 to 19 amino acid residues.

4. The peptide according to any one of the preceding claims, wherein the peptide is capable of binding to the SARS-CoV-2 Spike S1 protein.

5. The peptide according to any one of the preceding claims, wherein the peptide is capable of inhibiting the binding of the spike S1 protein of SARS-CoV-2 to the human ACE2 receptor.

6. The peptide according to any one of the preceding claims, wherein the peptide is further conjugated to a moiety.

7. The peptide according to any one of the preceding claims, wherein the peptide is further modified by glycosylation, phosphorylation, PEGylation, amidation, esterification, acylation, acetylation and/or alkylation.

8. A composition comprising the peptide according to any one of claims 1 to 7.

9. The composition according to claim 8, wherein the composition comprises one or more independently selected from each of adjuvants, excipients, carriers, and nutrients.

10. The peptide according to any one of claims 1 to 7, or the composition according to claim 8, for use as a medicament.

11. The peptide according to any one of claims 1 to 7, or the composition according to claim 8, for use in prevention and/or treatment of a SARS-CoV-2-related disease in a subject.

12. The peptide according to any one of claims 1 to 7, or the composition according to claim 8, for use according to claim 11, wherein the disease is COVID-19.

13. The peptide according to any one of claims 1 to 7, the composition according to claim 8, wherein the administration route is systemic, local, enteral, or parenteral.

## Patentansprüche

1. Peptid, wobei das Peptid die Aminosäuresequenz von SEQ ID NO: 1 umfasst oder eine Sequenzvariante davon, die sich durch einen oder zwei Aminosäurereste von der SEQ ID NO unterscheidet.

2. Peptid nach einem der vorhergehenden Ansprüche, wobei die eine oder zwei Aminosäuren der Sequenzvariante durch Substitution, Addition und/oder Deletion realisiert sind.

3. Peptid nach einem der vorhergehenden Ansprüche, wobei das Peptid im Bereich von 16 bis 50 Aminosäureresten, wie z. B. im Bereich von 16 bis 30 Aminosäureresten, wie z. B. im Bereich von 16 bis 22 Aminosäureresten, wie z. B. im Bereich von 16 bis 20 Aminosäureresten, wie z. B. im Bereich von 17 bis 19 Aminosäureresten umfasst.

4. Peptid nach einem der vorhergehenden Ansprüche, wobei das Peptid in der Lage ist, an das SARS-CoV-2-Spike-S1-Protein zu binden.

5. Peptid nach einem der vorhergehenden Ansprüche, wobei das Peptid in der Lage ist, Binden des Spike-S1-Proteins von SARS-CoV-2 an den menschlichen ACE2-Rezeptor zu hemmen.

6. Peptid nach einem der vorhergehenden Ansprüche, wobei das Peptid ferner an einen Teil konjugiert ist.

7. Peptid nach einem der vorhergehenden Ansprüche, wobei das Peptid ferner durch Glykosylierung, Phosphorylierung, PEGylierung, Amidierung, Veresterung, Acylierung, Acetylierung und/oder Alkylierung modifiziert ist.

8. Zusammensetzung, umfassend das Peptid nach einem der Ansprüche 1 bis 7.

9. Zusammensetzung nach Anspruch 8, wobei die Zusammensetzung einen oder mehrere unabhängig voneinander ausgewählte Hilfsstoffe, Arzneiträger, Trägerstoffe und Nährstoffe umfasst.

10. Peptid nach einem der Ansprüche 1 bis 7 oder Zusammensetzung nach Anspruch 8 zur Verwendung als ein Medikament.

11. Peptid nach einem der Ansprüche 1 bis 7 oder Zusammensetzung nach Anspruch 8 zur Verwendung bei der Prävention und/oder Behandlung einer SARS-CoV-2-bezogenen Krankheit in einem Subjekt.

12. Peptid nach einem der Ansprüche 1 bis 7 oder Zusammensetzung nach Anspruch 8 zur Verwendung nach Anspruch 11, wobei die Krankheit COVID-19 ist.

13. Peptid nach einem der Ansprüche 1 bis 7, Zusammensetzung nach Anspruch 8, wobei der Verabreichungsweg systemisch, lokal, enteral oder parenteral ist.

## Revendications

1. Peptide, dans lequel le peptide comprend la séquence d'acides aminés de SEQ ID NO: 1, ou une variante de séquence de celle-ci différant d'un ou deux résidus d'acides aminés de ladite SEQ ID NO.

2. Peptide selon l'une quelconque des revendications précédentes, dans lequel l'un ou les deux acides aminés de la variante de séquence sont réalisés par substitution, addition et/ou délétion.

3. Peptide selon l'une quelconque des revendications précédentes, dans lequel le peptide comprend dans la plage de 16 à 50 résidus d'acides aminés, telles que dans la plage de 16 à 30 résidus d'acides aminés, telles que dans la plage de 16 à 22 résidus d'acides aminés, telles que dans la plage de 16 à 20 résidus d'acides aminés, telles que dans la plage de 17 à 19 résidus d'acides aminés.

4. Peptide selon l'une quelconque des revendications précédentes, dans lequel le peptide est capable de se lier à la protéine Spike S1 du SARS-CoV-2.

5. Peptide selon l'une quelconque des revendications précédentes, dans lequel le peptide est capable d'inhiber la liaison de la protéine Spike S1 du SARS-CoV-2 au récepteur ACE2 humain.

6. Peptide selon l'une quelconque des revendications précédentes, dans lequel le peptide est également conjugué à une fraction.

7. Peptide selon l'une quelconque des revendications précédentes, dans lequel le peptide est également modifié par glycosylation, phosphorylation, pégylation, amidation, estérification, acylation, acétylation et/ou alkylation.

8. Composition comprenant le peptide selon l'une quelconque des revendications 1 à 7.

9. Composition selon la revendication 8, dans laquelle la composition comprend un ou plusieurs éléments choisis indépendamment parmi chacun des adjuvants, excipients, supports et nutriments.

10. Peptide selon l'une quelconque des revendications 1 à 7, ou composition selon la revendication 8, destinés à être utilisés comme médicament.

11. Peptide selon l'une quelconque des revendications 1 à 7, ou composition selon la revendication 8, destinés à être utilisés dans la prévention et/ou le traitement d'une maladie liée au SARS-CoV-2 chez un sujet.

12. Peptide selon l'une quelconque des revendications 1 à 7, ou composition selon la revendication 8, destinés à être utilisés selon la revendication 11, dans lesquels la maladie est la COVID-19.

13. Peptide selon l'une quelconque des revendications 1 à 7, ou composition selon la revendication 8, dans lesquels la voie d'administration est systémique, locale, entérale ou parentérale.
